# EUROPÄISCHE PATENTSCHRIFT

(11) **EP 2 120 704 B1**
(45) Veröffentlichungstag und Bekanntmachung des Hinweises auf die Patenterteilung: **16.01.2013**
(21) Anmeldenummer: 07856185.9
(22) Anmeldetag: 20.11.2007
(51) Int. Cl.: A61B 8/00, G01R 33/563, A61B 5/055, G01R 33/48

(54) **MR-BILDKONTRAST MITTELS ULTRASCHALL**
MR IMAGE CONTRAST BY MEANS OF ULTRASOUND
CONTRASTE D'IMAGE MR PAR ULTRASON

(30) Priorität: 08.12.2006 DE 102006058264
(43) Veröffentlichungstag der Anmeldung: 25.11.2009
(73) Patentinhaber: Rheinische Friedrich-Wilhelms-Universität Bonn, 53113 Bonn (DE)
(72) Erfinder: MAIER, Karl-Georg, 53119 Bonn (DE)
(74) Vertreter: Viering, Jentschura & Partner
(86) Internationale Anmeldenummer: PCT/EP2007/010002
(87) Internationale Veröffentlichungsnummer: WO 2008/067905

(56) Entgegenhaltungen:
- US-A- 5 307 812
- US-B1- 6 246 895
- MUTHUPILLAI R ET AL: "MAGNETIC RESONANCE ELASTOGRAPHY BY DIRECT VISUALIZATION OF PROPAGATING ACOUSTIC STRAIN WAVES" SCIENCE, AMERICAN ASSOCIATION FOR THE ADVANCEMENT OF SCIENCE,, US, Bd. 269, Nr. 5232, 29. September 1995 (1995-09-29), Seiten 1854-1857, XP000560135 ISSN: 0036-8075
- SACK I ET AL: "Magnetic resonance elastography and diffusion-weighted imaging of the sol/gel phase transition in agarose" JOURNAL OF MAGNETIC RESONANCE, ACADEMIC PRESS, ORLANDO, FL, US, Bd. 166, Nr. 2, Februar 2004 (2004-02), Seiten 252-261, XP004483633 ISSN: 1090-7807

## Beschreibung

Die Erfindung richtet sich auf ein Verfahren zur Erzeugung eines Kontrast gebenden Effektes in der Bilddarstellung eines Magnetresonanztomographie-Verfahrens und/oder -systems, bei welchem weiche Materie dem Einfluss eines Hochfrequenzpulsfeldes einer Magnetresonanztomographieanlage ausgesetzt und in das Hochfrequenzpulsfeld Ultraschallsignale gepulst eingekoppelt und eingestrahlt werden. Ferner richtet sich die Erfindung auf die Verwendung einer Magnetresonanztomographieanlage. Weiterhin richtet sich die Erfindung auf eine einen Ultraschallemitter umfassende Vorrichtung sowie eine Magnetresonanztomographieanlage mit einer solchen Vorrichtung.

Der Effekt der kernmagnetischen Resonanz oder NMR (Nuclear Magnetic Resonance) wird seit langer Zeit vielfältig genutzt. In der Chemie, Biochemie und Biologie wird sie vor allem zur Strukturuntersuchung von Molekülen verwendet, in der Physik zur Untersuchung von Festkörpereigenschaften und insbesondere findet sie in der Medizin als Bild gebendes Verfahren zur Darstellung von Strukturen im Inneren des Körpers Anwendung. Zur bildlichen Darstellung von Körpern wird das so genannte MRI (Magnetic Resonance Imaging) im Rahmen der Magnetresonanz (MR) oder der Magnetresonanztomographie (MRT) angewendet. Ein bekannter synonymer Begriff für die Magnetresonanztomographie ist die Kernspintomographie.

Bei der NMR wird der Effekt ausgenutzt, dass sich die Kernspins eines Mediums oder eines Materials in einem äußeren Magnetfeld zu einem etwas größeren Anteil parallel zu diesem Magnetfeld ausrichten als antiparallel dazu. Mittels hochfrequenter Felder, den so genannten HF(Hochfrequenz)-Pulsen, kann dieser Gleichgewichtszustand gestört werden. Die daraus entstehende präzedierende Quermagnetisierung lässt sich in Form eines hochfrequenten Induktionssignals beobachten (FID = Free Induction Decay bzw. Hahn-Echo). Zu diesem Signal tragen alle in einem beobachteten Messvolumen detektierten Protonen bei. Jedes Gewebe lässt sich durch charakteristische Zeitkonstanten beschreiben, die ein Maß für die Dauer bis zur Rückkehr in den Gleichgewichtszustand des in dem hochfrequenten Feld befindlichen Gewebes darstellen. Diese Zeitkonstanten werden als T₁- und T₂-Relaxationszeit bezeichnet. Eine weitere gewebespezifische Größe, die bei der Bildgebung dieser Bild gebenden Verfahren MRT/MRI eine Rolle spielt, ist die Protonendichte. Diese drei Kontrast gebenden Effekte, nämlich die Protonendichte, die so genannte Spin-Gitter-Relaxation (T₁) und die Spin-Spin-Relxation (T₂) werden genutzt, um Gewebebereiche mit unterschiedlichen Eigenschaften durch einen Kontrastunterschied in der Bilddarstellung der Bild gebenden Verfahren zu nutzen. Für jede dieser drei vorstehenden Größen gibt es spezielle Pulssequenzen, die zu einem gut darstellbaren Kontrast führen. Sollen beispielsweise zwei Gewebe oder Gewebebereiche dargestellt werden, die ähnliche Relaxationszeiten aufweisen, sich aber in der Protonendichten deutlich unterscheiden, so wird zur Datengewinnung eine Pulssequenz benutzt, die zu einem guten Kontrast bei unterschiedlicher Protonendichte führt. Probleme treten allerdings dann auf, wenn sich zwei Gewebe oder Gewebebereiche in allen drei vorstehenden Größen nur geringfügig unterscheiden. Dann lassen sich diese Gewebebereiche nur dadurch unterscheiden, dass von außen Kontrast gebende Mittel zugegeben werden. Beispielsweise kann im medizinischen Bereich durch die gewebespezifische Zugabe von Molekülen mit einem ungepaarten Elektronenpaar, die als paramagnetische Substanzen bezeichnet werden, die T₁-Zeit stark verringert werden.

Eine andere, komplementäre Technik stellt die NAR (Nuclear Acoustic Resonance) dar, die bei der Untersuchung bestimmter Metalle oder Legierungen, die für elektromagnetische Wellen der NMR aufgrund des Skineffektes schlecht durchlässig, aber für Ultraschall problemlos durchlässig sind, Anwendung findet. Bei der NAR-Methode erfolgt eine direkte Ankopplung einer akustischen Welle an das Kernspinsystem. Auch hierdurch lässt sich die T₁-Zeit verkürzen. Während bei der NMR die Übergänge zwischen den Kernspin-Energieniveaus durch Ankopplung des magnetischen Kernmomentes an äußere RF-Magnetfelder als Absorption oder Dispersion elektromagnetischer Strahlung induziert werden, geschieht dies im Fall der NAR durch Wechselfelder, welche durch den Ultraschall eingebracht werden. NAR-Untersuchungen wurden bisher an Festkörpern bevorzugt mit großem Kernquadropolmoment durchgeführt.

Aus der US 6,246,895 B1 ist nun ein Verfahren bekannt, bei welchem mithilfe eines MRI-Systems das Bild einer in einer weichen Materie, in diesem Fall Agar-Gel, ausgebildeten Schallwelle, die sich in dem Hochfrequenzpulsfeld des Tomographen befindet und von einer Ultraschallwellen erzeugenden Ultraschallquelle beschallt wird, dargestellt wird. Hierbei wird die Probe einem extrem starken magnetischen Wechselfeldgradienten ausgesetzt, über den die periodische Auslenkung durch den Ultraschall phasenkodiert wird. Die Frequenz des magnetischen Wechselfeldes und die des Ultraschalls müssen in diesem Verfahren identisch sein. Ausgelesen wird diese Information mit Hilfe der mathematischen Dekodierung einer Ortskoordinate, welche somit für die Tomographie auf diesem Wege nicht mehr zur Verfügung steht. Ultraschallfelduntersuchungen sind mit dieser Methode möglich, ein Transfer auf lebendes Gewebe ist aufgrund des hohen magnetischen Wechselfeldes und der hohen Ultraschallamplitude nicht möglich.

Eine weitere Anwendung der Kombination von Magnetresonanz und Ultraschall besteht gemäß der WO 01/43640 A2 darin, die in einem Magnetresonanztomographen sowohl für ein MRI-Verfahren als auch für ein Ultraschall-Verfahren vorhandenen Sende-, Empfangs- und Auswerteeinheiten so miteinander zu verknüpfen, dass in der bildlichen Darstellung des Magnetresonanztomographen sowohl die verarbeiteten NMR-Signale als auch die verarbeiteten Ultraschall-Signale sichtbar gemacht werden. Auch in diesem Fall ist eine gegenseitige Beeinflussung der Signale oder eine Beeinflussung auf die Bildqualität nicht ersichtlich.

Der Erfindung liegt demgegenüber die Aufgabe zugrunde, eine Lösung zu schaffen, die eine Einflussnahme auf den Kontrast der bildlichen Darstellung eines Bild gebenden MRT-Verfahrens ermöglicht.

Bei einem Verfahren der eingangs bezeichneten Art wird diese Aufgabe erfindungsgemäß dadurch gelöst, dass eine Ultraschallsignalpulssequenz derart in eine von der Magnetresonanztomographieanlage erzeugte Bildpulsfolge oder - sequenz eingekoppelt und eingestrahlt wird, dass in der Bilddarstellung der Magnetresonanztomographieanlage die durch Ultraschallstrahlungsdruck induzierte Bewegung der weichen Materie als Bildkontrast darstellbar und/oder dargestellt wird.

Die Erfindung geht also von der Erkenntnis aus, dass mittels gepulsten Ultraschalls eine Kopplung des Schalls an das Kernspinsystem möglich ist und dies in Überlagerung mit und unter Beeinflussung der Hochfrequenzpulse des Magnetresonanztomographen bzw. eines NMR-Systems zu einer durch die Ultraschallstrahlung beeinflussten geänderten Bilddarstellung des bildgebenden MR- oder MRT- Verfahrens führt. Anders als beim Stand der Technik wird also nicht lediglich eine das Hochfrequenzpulsfeld nicht beeinflussende Ultraschallwelle dargestellt, sondern es wird eine aktive Beeinflussung des in dem Magnetfeld einem Hochfrequenzpuls ausgesetzten untersuchten Materials oder Materie vorgenommen und dann unmittelbar in der Bilddarstellung des MRT sichtbar gemacht. Diese Beeinflussung rührt daher, dass es durch die durch die Ultraschallstrahlung eingebrachte Dynamik zu einer Signalabschwächung kommt. Hierdurch sind bei den NMR-Verfahren bzw. dem Magnetresonanztomographie-Verfahren dank des Ultraschalls nun erhöhte Aufnahmefrequenzen möglich. Dies führt nicht nur zu kürzeren Aufnahmezeiten, sondern auch zu einer besseren zeitlichen Auflösung, der in der untersuchten Materie unter Einfluss der NMR stattfindenden Abläufe in der bildlichen Darstellung. Es wird nun die Sichtbarmachung der induzierten Bewegung der Materie durch Ultraschallstrahlung möglich. Dies führt zu Kontrastdarstellungen in dem Bild, die dann deutlich Ultraschall beschallte Materiebereiche von nicht Ultraschall beschallten Materiebereichen unterscheiden bzw. unterscheidbar machen. Durch die Ultraschallstrahlung wird in das der Magnetresonanztomographie ausgesetzte Material oder Medium eine Kollektivbewegung aufgrund eines viskosen Impulsübertrags eingebracht, wobei die Viskosität der jeweiligen weichen Materie oder Flüssigkeit die Reichweite und Art des Ultraschalleinflusses stark beeinflusst. Bereiche unterschiedlicher Viskosität zeigen nun unterschiedliche Kollektivbewegungen, die bei einer NMR-Messung in der Bilddarstellung durch unterschiedliche Kontraste dargestellt werden. Mit der Erfindung ist es somit möglich, über die bisherigen zur Kontrastgebung verwendeten Effekte hinaus die Viskosität eines der Magnetresonanztomographie unterworfenen Mediums oder unterschiedlicher Medienbereiche durch gezieltes Einstrahlen von Ultraschall während der Pulssequenz zur Bildgebung als Kontrast gebenden Effekt zu nutzen. Aufgrund der Krafteinwirkung auf das untersuchte Materievolumen bzw. das dargestellte Materievolumen kann die darzustellende Region zum Beispiel über die Bewegungsempfindlichkeit der Pulssequenz im Magnetresonanztomographen abgebildet werden. Bisher nicht darstellbare Gewebeparameter, wie beispielsweise die Viskosität, werden damit im Bild sichtbar. Die erfindungsgemäße zusätzliche Diagnosemöglichkeit kann in jedem Kernspintomographen eingesetzt werden. Die sonstigen Möglichkeiten der Kernspintomographie mit allen Variationen des Bildkontrastes, wie Protonendichte, T₁-/T₂-Wichtung, Diffusion Tensor Imaging, BOLD-Effekt für funktionelle Bildgebung, Elastographie, verschiedene Kontrastmittel, Spektroskopie über chemische Verschiebung, Temperaturmessung bleiben weiterhin möglich.

Insgesamt besteht die Erfindung in der Sichtbarmachung des viskosen Impulsübertrages oder Schallweges oder Ultraschallstrahlungsdruckes in einer weichen Materie durch Einkopplung eines, insbesondere abgeschirmten, Ultraschallemitters in das Hochfrequenzfeld eines Kernspintomographen unter Anpassung oder zumindest Berücksichtigung der Bewegungsempfindlichkeit des Tomographenbildes bzw. dessen Bildsequenzen.

In Ausgestaltung sieht die Erfindung daher vor, dass der Schallweg und/oder der Ultraschallstrahlungsdruck und/oder ein schallinduzierter Impulsübertrag in der weichen Materie dargestellt wird.

Besonders zweckmäßig lassen sich zur Abbildung der gerichteten Bewegung diffusionssensitive Sequenzen verwenden, so dass die Erfindung auch vorsieht, dass die Sequenzen der Hochfrequenzpulse diffusionssensitive Sequenzen sind.

Erfindungsgemäß ist es auch möglich, dass die Bildpulsfolge oder -sequenz (Echosequenz) um einen weiteren bipolaren Feldgradienten, insbesondere zwischen dem 90°- und dem 180°-Puls erweitert wird.

Besonders günstig lässt sich der Kontrast gebende Effekt dadurch erzielen, dass die Ausbreitungsrichtung des Ultraschalls parallel zum diffusionssensitiven Gradienten gewählt wird, was die Erfindung in weiterer Ausgestaltung ebenfalls vorsieht.
Hierbei ist es von besonderem Vorteil, wenn längs eines Gradienten, vorzugsweise eines Gradienten in Richtung des Hauptmagnetfeldes B₀, eine Schicht selektiert-und in der selektierten Schicht in den verbleibenden Richtungen, vorzugsweise in y- und x-Richtung, über die Phase und die Frequenz eine Punktselektion durchgeführt wird, wobei der Ultraschallimpuls jeweils nach der Schichtselektion eingestrahlt wird, was die Erfindung auch vorsieht.

Besonders vorteilhafte Ergebnisse zur Erzeugung des Kontrast gebenden Effektes werden dann erreicht, wenn Ultraschall mit einer Frequenz zwischen 2 und 30 MHz und einer Pulslänge zwischen 1 und 50 ms und einem Schalldruck von ≤ 1 bar eingekoppelt und eingestrahlt wird.

Um in besonders günstiger Weise eine Ultraschallquelle in dem Bereich eines Magnetresonanztomographen anordnen zu können, sieht die Erfindung weiterhin vor, dass ein abgeschirmter Ultraschallemitter und/oder -transmitter innerhalb der zentralen Öffnung eines Magnetresonanztomographen angeordnet und in dessen Hochfrequenzpulsfeld eingekoppelt wird.

Hierbei kann weiterhin vorgesehen sein, dass Ultraschall über einen Wellenleiter in das Hochfrequenzpulsfeld der Magnetresonanztomographieanlage eingekoppelt wird.

Besonders günstig lässt sich die Einkopplung dadurch erreichen, dass der Ultraschallemitter und/oder -transmitter mittels einer Hochfrequenzantenne mit dem Hochfrequenzpulsfeld synchronisiert gekoppelt wird. Hierbei kann weiterhin vorgesehen sein, dass mit der Hochfrequenzantenne das Hochfrequenzpulssignal des Magnetresonanztomographen detektiert und für die gezielte Einstrahlung des Ultraschallimpulses genutzt wird.

Zweckmäßigerweise wird beim erfindungsgemäßen Verfahren zur zeitlichen Positionierung des Ultraschalls in der bildgebenden Sequenz ein Steuersignal der Magnetresonanztomographieanlage genutzt.

Eine für medizinische Untersuchungszwecke besonders günstige Positionierung der Ultraschallquelle ist dann gegeben, wenn der Ultraschallemitter und/oder -transmitter im Bereich einer Hochfrequenzspule innerhalb des Magnetresonanztomographen angeordnet wird oder der Ultraschall mittels eines Wellenleiters in diesen Bereicht transportiert wird. Die Hochfrequenzspule ist insbesondere diejenige, die zur Aussendung der Hochfrequenzpulse verwendet wird.

Die oben stehende Aufgabe wird weiterhin gelöst durch die Verwendung einer Magnetresonanztomographieanlage durch Einbringung eines abgeschirmten Ultraschallemitters in die zentrale Öffnung eines Magnetresonanztomographen und Einkopplung des Ultraschallemitters in das Hochfrequenzpulsfeld des Magnetresonanztomographen sowie zeitlich abgestimmtes Einstrahlen von Ultraschallpulsen in das von der Magnetresonanztomographieanlage erzeugte Hochfrequenzpulsfeld zur Erzeugung einer kontrastreichen Darstellung der in einer in das Untersuchungsfeld des Tomographen eingebrachten weichen Materie erzeugten durch Ultraschallstrahlung induzierten Bewegung der Materie in der Bilddarstellung der Magnetresonanztomographieanlage.

Weiterhin wird die oben stehende Aufgabe erfindungsgemäß durch eine Vorrichtung gelöst, die einen elektromagnetisch abgeschirmten Ultraschallemitter mit Mitteln zur Detektion eines bildgebend genutzten Hochfrequenzpulsfeldes eines mit dem Ultraschallemitter in Wirkverbindung stehenden Magnetresonanztomographen und mit Mitteln zur synchronisierten Einstrahlung von Ultraschallpulssequenzen in dessen Bildpulsfolge oder -sequenz umfasst. Mit einer solchen Vorrichtung ist es möglich, auch bestehende Magnetresonanztomographen hardwaretechnisch nachzurüsten. Diese apparative Vorrichtung lässt sich in jeden Tomographen unkompliziert einbauen. Es ist kein Eingriff, weder in die Hardware noch in die Software des Kernspintomographen, notwendig. Die zeitliche Synchronisation erfolgt dabei über ein von einer Antenne der Vorrichtung empfangenes Antennensignal, das das Hochfrequenzstreufeld außerhalb des Tomographen auffängt. Die notwendige Zeitsteuerung der Pulse und die Ultraschallpulserzeugung erfolgt in einer separaten Elektronik, wobei die einzige Verbindung am Ultraschallkopf im Tomographen ein nicht magnetisches Koaxialkabel ist.

Schließlich sieht die Erfindung auch noch eine Magnetresonanztomographieanlage vor, die eine Vorrichtung nach Anspruch 14 sowie Mittel zur Darstellung einer gerichteten, durch Ultraschallstrahlungsdruck induzierten bewegung in einem in dem Magnetresonanztomographen der Magnetresonanztomographieanlage dem Hochfrequenzpulsfeld ausgesetzten Volumenelement aus weicher Materie.

Unter weicher Materie wird biologisches Material, menschliches oder tierisches Gewebe, Flüssigkeit und sonstige nicht harte Materie verstanden, die kernspintomographenfähig ist. Unter Ultraschall werden Frequenzen zwischen 2 x 10⁴ bis 1 x 10⁹ Hz verstanden.

Die Erfindung ist nachstehend anhand eines Ausführungsbeispieles näher erläutert. Die Erläuterung erfolgt im Zusammenhang mit Figuren. Hierbei zeigt
- Figur 1: in schematischer Darstellung einen Kernspintomographen mit zugeordneten Einrichtungen zur Ultraschalleinstrahlung,
- Figur 2: in schematischer Darstellung die Einrichtungen zur Ultraschalleinstrahlung,
- Figur 3: in schematischer Schnittdarstellung die Emitter- und Probenbehälteranordnung,
- Figur 4: in schematischer Darstellung die Einrichtungen zur Einkopplung der HF-Antenne,
- Figur 5a, 5b: eine Aufstellung möglicher MR-Sequenzen,
- Figur 6: in schematischer Darstellung die ortskodierenden Schritte in einer Hahn-Echo-Frequenz,
- Figur 7: in schematischer Darstellung eine Hahn-Echo-Sequenz mit Ultraschalleinstrahlung,
- Figur 8: in schematischer Darstellung den Einfluss eines bipolaren Gradienten,
- Figur 9: Bilddarstellungen unter Einfluss einer bewegungsempfindlichen NMR-Sequenz,
- Figur 10: Bilddarstellungen unter Einfluss einer T₂ - gewichteten Sequenz,
- Figur 11: Bilddarstellungen unter Einfluss einer T₁ - gewichteten Sequenz und in
- Figur 12: Bilddarstellungen des Ultraschalleinflusses auf eine weiche Materie.

Die charakteristischen Größen einer Schallwelle Schallauslenkung, Schallschnelle und Schalldruck pflanzen sich periodisch in einem Medium fort. In realen Körpern gibt es immer eine Dämpfung, das heißt einen Energietransfer von der Schallwelle ins Medium, so dass die Schallintensität mit dem Abstand von der Schallquelle immer kleiner wird. In weicher Materie oder auch "soft tissue", worunter hier insbesondere biologisches Gewebe, menschliches Gewebe, tierisches Gewebe, Zellen, biologisches Material, Agar, Flüssigkeiten, Gel, oder ähnliches verstanden wird, das "kernspintomographenfähig", d.h. mit dieser Methode bildlich darstellbar ist, und teilweise Wasser enthält, wird die Dämpfung auch durch die Viskosität bestimmt und man spricht von einem viskosen Impulsübertrag. Auf diese Weise gibt eine Ultraschallwelle einen kleinen Teil (Dämpfung) ihres Impulses mit jeder Verdichtung an das jeweilige Medium ab. Dieser Impulsteil zeigt zunächst in Richtung der Bewegung und wird dann über Folgestöße in alle Richtungen aufgeteilt. Da der Gesamtimpuls erhalten bleibt, entsteht eine gerichtete Bewegung, deren Geschwindigkeit mit der Anzahl der Schwingungen zunimmt bis sich ein Gleichgewicht mit der Reibungskraft einstellt. Die Reibungskraft in Flüssigkeiten ist direkt proportional zum Produkt aus Geschwindigkeit und Viskosität. Ein weiterer Grund für eine Fließbewegung ist, dass die Verdichtung durch den sich ausdehnenden Schallwellenleiter (das jeweilige Medium, im erfindungsgemäßen Fall die weiche Materie) eine vorgegebene Richtung besitzt, während die Dekompression aufgrund des Zusammenziehens des jeweiligen Mediums (weiche Materie) aus allen Richtungen stattfindet.
Diese Bewegung bezieht sich jedoch nur auf das Volumen, welches direkt durch den Hub (einige nm) am Schallleiter betroffen ist und wirkt sich nicht auf die weiter entfernten Bereiche aus. Da die durch den Impulsübertrag in die Flüssigkeit eingebrachte Bewegung mitverantwortlich für die Signalreduktion bei der NMR (NMR = Nuclear Magnetic Resonance oder kernmagnetische Resonanz) ist, hängt der Einfluss von der Viskosität ab. Bei Messungen an Flüssigkeiten, bei denen es sich um Mischungen aus Wasser und Glycerin in einem Mischungsverhältnis von 2:1 bzw. 1:1 handelt, wurde mit einer Hahn-Echo-Sequenz, bei der der Zeitpunkt der Ultraschalleinstrahlung bezogen auf den 90°-Puls variiert wurde, festgestellt, dass der Einfluss auf die Signalamplitude bei einer höheren Viskosität kleiner ist, so dass die eingebrachte Bewegung bei höherer Viskosität schneller abklingt. Durch die hochfrequente mechanische Einwirkung des Ultraschalls auf eine Flüssigkeit wird die Magnetisierung beeinflusst. Auf diese Weise nehmen Ultraschallwellen Einfluss auf das Kernspinensemble. Hierbei zeigt die Einflussstärke auf die T*₂ - Zeit keine starke Frequenzabhängigkeit. Die Einflussstärke skaliert mit der in das System eingebrachten Ultraschallenergie und sie hängt mit der Viskosität zusammen. Durch den Ultraschall wird ein Prozess angestoßen, der für eine gewisse Zeit über die Dauer des Ultraschallpulses hinaus bestehen bleibt. Es kommt zu einer kollektiven Bewegung in der Probe aus weicher Materie im mikrometer Bereich, im Gegensatz zu den nur nanometer großen Schallauslenkungen. Dies wurde ortsaufgelöst in einem 1.5 T(Tesla)-Kernspintomographen oder Magnetresonanztomographen, wie er in der Medizin verwendet wird, untersucht. Hierbei sind die Begriffe Kernspintomograph und Magnetresonanztomograph Synonyme.

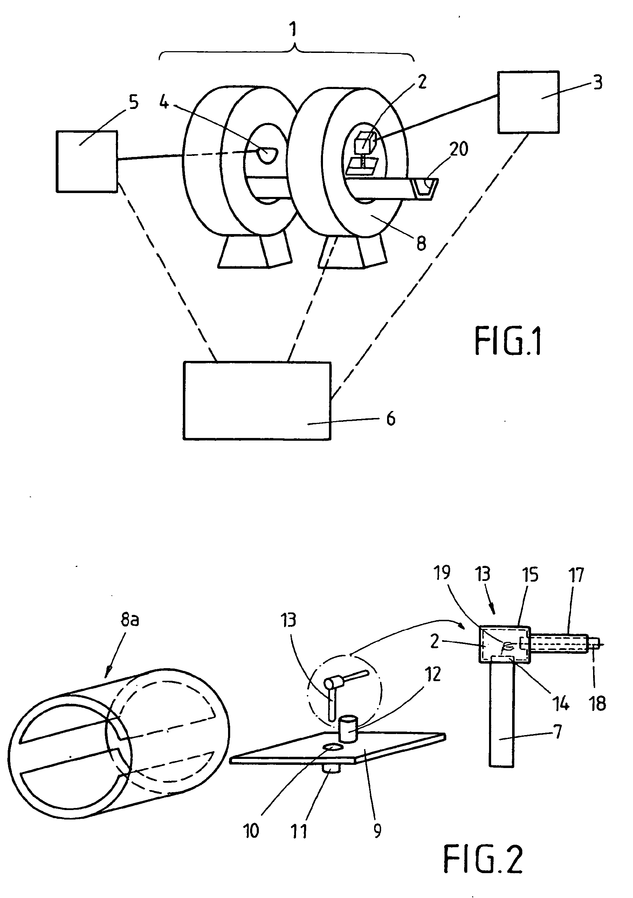

Die verwendete Magnetresonanztomographieanlage umfasst im Wesentlichen einen dreiteiligen Aufbau, aus einem üblichen standardmäßigen 1,5T Kernspintomograph, der in der Figur 1 schematisch dargestellt und mit dem Bezugszeichen 1 versehen ist, einem Ultraschallemitter und/oder -transmitter 2 mit seiner Ansteuerelektronik 3 und einem Einkopplungselement 4, im vorliegenden Fall eine Hochfrequenz (HF) - Antenne, mit angeschlossener Schaltung 5 zur Ein- und Ankopplung des vom Ultraschallemitter ausgestrahlten Ultraschalls in das Hochfrequenzpulsfeld des Kernspintomographen 1 und zur Synchronisation von Ultraschall und Tomograph 1. Gesteuert werden die Anlage und das Verfahren durch Steuereinheiten und -elemente 6. die Hochfrequenz - oder Radiofrequenz - Generatoren und - Verstärker umfassen. Der verwendetet Ultraschalltransmitter 2 ist derart dimensioniert, dass nicht nur der Wellenleiter 7 sondern der als Piezokeramik 9 ausgebildete Emitter, und somit der Ultraschalltransmitter und -emitter 2 als Ganzes bzw. als eine Einheit in die Sende - und Empfangsspule 8 des Kernspintomographen 1 eingebaut werden kann, wie aus der Figur 1 ersichtlich ist. Es ist aber auch möglich, den Schall auch von außen über geeignete Wellenleiter zum Medium zu führen, was im vorliegenden Ausführungsbeispiel allerdings nicht realisiert ist.. Die Sende- und Empfangsspule 8 ist z.B. eine Sattelspule, wie sie in der medizinischen Anwendung eines Kernspintomographen 1 für Aufnahmen am Patientenkopf benutzt wird.

Der schematische Aufbau für die Messungen mit Ultraschall an einem Kernspintomographen 1 ist in der Figur 2 schematisch dargestellt. Die Figur 2 zeigt im linken Teilbild eine Sattelspule 8a (Kopfspule), im mittleren Teilbild eine Basisplatte 9 mit einer Bohrung 10 für den Probenbehälter 11 und einem Stativ 12 als Halterung für den Ultraschallkopf 13 bzw. den Ultraschalltransmitter und -emitter 2. Die Basisplatte 9, der Probenbehälter 11 und das Stativ 12 bestehen vollständig aus Teflon, da dies nur geringe elektrische Verluste mit sich bringt sich keine Protonen im Aufbau dieser Anordnung befinden, so dass diese Anordnung für den Kernspintomographen 1 "unsichtbar" ist. Im rechten Teilbild ist der schematische Aufbau eines Ultraschallkopfes 13 mit dem Wellenleiter 7 als Ultraschalltransmitter, einer Piezokeramik 14 als Ultraschallemitter und einem Gehäuse 15 dargestellt. Die Sattelspule hat eine Abmessung von etwa 20 x 20 cm, woran die Basisplatte 9 mit ihrer Seitenlänge von 20 cm angepasst ist. Die sehr dünne, runde 9,45 MHz Piezokeramik 14 weist einen Durchmesser von ca. 5 mm auf und ist innerhalb des Gehäuses 15 auf eine 2 mm starke Innenwandfläche aufgeklebt und an den koaxial auf der gegenüberliegenden Wandfläche des Gehäuses 15 außen angeklebten Wellenleiter 7 aus Quarz angekoppelt. Der als Quarzstab ausgebildete Wellenleiter 7 weist einen Durchmesser von 5 mm und eine Länge von 40 mm auf. Das Gehäuse 15 ist vollständig aus Metall einer Cu-Al-Legierung hergestellt, so dass es eine elektromagnetische Hochfrequenz (HF) - Abschirmung für die Piezokeramik 14 ausbildet. Die Grundfläche des quaderförmigen Gehäuses 15 beträgt 10 x 10 mm. Diese Konstruktion bringt zwei entscheidende Vorteile mit sich. Die Piezokeramik 14 ist elektromagnetisch gegenüber dem NMR-Schwingkreis isoliert und außerdem kann aufgrund des Kontaktes zum Aluminiumgehäuse 15 sehr gut Wärme abgeführt werden. Dies ermöglicht eine höhere Leistung im Betrieb. Die Verluste in der Schallintensität bei den Übergängen Keramik-Aluminium und Aluminium-Quarz sind sehr klein, da zum einen die Impedanzunterschiede zwischen Keramik - Aluminium und Aluminium - Quarz klein sind, und zum anderen die Klebeschichten dünn gegenüber der Wellenlänge sind, womit eine gute Transmission gewährleistet ist. Eine weitere empfindliche Stelle für elektromagnetische Lecks am Transmitter stellt die Zuleitung 18 dar. Diese ist innerhalb des Aluminiumrohrs 17 bis zum Transmitter geführt und ist somit auch für den NMR-Schwingkreis "unsichtbar".

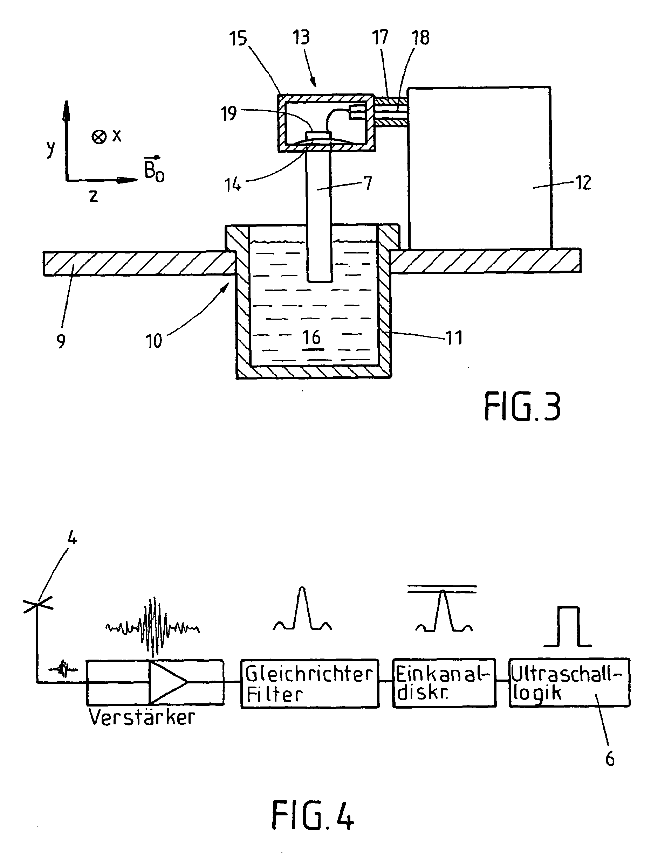

In der Basisplatte 9 befindet sich zentral die Bohrung 10, in die die zu untersuchende und in der Bilddarstellung des Kernspintomographen 1 darzustellende weiche Materie 16, im vorliegenden Fall eine wasserhaltigen Flüssigkeit, mit Hilfe des sie umgebenden zylindrischen Probenbehälters aus Teflon 11 eingebracht ist, wie dies aus der vergrößerten und schematischen Schnittdarstellung der Figur 3 ersichtlich ist. Außerhalb der Öffnung 10 ist auf der Basisplatte 9 das Stativ 12 aus Teflon angebracht und über ein ebenfalls aus einer Cu-Al-Legierung bestehendes Rohr 17, das ein innerhalb verlaufendes Koaxial - Anschlusskabel 18 elektromagnetisch gegen Hochfrequenz von außen abschirmt, mit dem Ultraschallkopf 13 verbunden. An das Anschlusskabel 18 ist eine Hochfrequenz angeschlossen, die über das Anschlusskabel 18 und eine Spule 19 zur Blindleistungskompensation auf die Piezokeramik 14 gegeben wird. Die mit dem Stativ 12 und dem Ultraschallkopf 13 versehene Basisplatte 9 wird zentral in der Mitte der Kopfspule 8a eingebaut. Die Kopfspule 8a mit Basisplatte 9, weichem Material 16 im Probenbehälter 11 und Schallgeber bzw. Schallkopf 13 wird - wie bei medizinischen Untersuchungen ansonsten für den Patienten üblich - mit der Patientenbahre 20 in die Mitte des Kernspintomographen 1 gefahren, wo die Messungen durchgeführt werden.

Die Steuer- und Ansteuerelektronik 3 des Ultraschalltransmitters und -emitters 2 umfasst eine Triggereinheit, einen Pulsgenerator, einen HF - Generator und einen Verstärker. Die Breite und die Position des jeweiligen Ultraschall - Pulses innerhalb der jeweils während der Durchführung des erfindungsgemäßen Verfahrens verwendeten Sequenz werden an der Triggereinheit eingestellt. Die Amplitude wird mittels des Generators und des Verstärkers erzeugt. Der Frequenzgenerator der Ultraschall - Ansteuerelektronik 3 wird durch einen weiteren Kanal der Triggereinheit extern getriggert. Auf diese Weise können die Breite des Ultraschallpulses und seine Position innerhalb der jeweiligen Sequenz beliebig variiert werden. Die Amplitude des Ultraschallpulses wird am Frequenzgenerator eingestellt. Da die maximale Amplitude bei dem Ausführungsbeispiel lediglich ca. 10 Vₛₛ an 50 Ohm beträgt, wird das Signal durch den Verstärker auf etwa 40 Vₛₛ verstärkt. es können aber auch weit höhere bzw. größere Amplituden eingestellt oder verwendet werden. Das verstärkte Signal wird dann auf den Ultraschallemitter 2 und von dort auf den Wellenleiter 7 gegeben.

Zur zeitlichen Positionierung des Ultraschalls bzw. der Ultraschallpulse in den entsprechenden NMR-Pulsen des Kernspintomographen 1 wird zusätzlich die Hochfrequenzantenne 4, mit der die Hochfrequenzsignale (63 MHz bei 1.5 T) des Kernspintomographen 1 detektiert werden, auf der Patientenliege 20 außerhalb des Tomographenmagneten befestigt. Die Signale, die von der Antenne 4 detektiert werden, werden in einem ersten Schritt mit Breitbandverstärkern verstärkt. Die 90°- und 180°-Pulse liegen dadurch deutlich über dem Rauschen.

In einem zweiten Schritt werden die Hochfrequenzsignale dann über einen Gleichrichter und Filter weitestgehend auf ihre Einhüllende reduziert und dann mit einem Einkanaldiskriminator in ein Triggersignal an die Impulslogik für die Steuerung 3, 6 des Ultraschalls umgewandelt, wie dies schematisch in Figur 4 dargestellt ist. Mit diesem schematischen Aufbau zur Detektion der Hochfrequenzpulse des Kernspintomographen 1 ist ein mit diesen synchronisiertes Einkoppeln und Einstrahlen des Ultraschalls in das Hochfrequenzpulsfeld des Kernspintomographen 1 möglich.

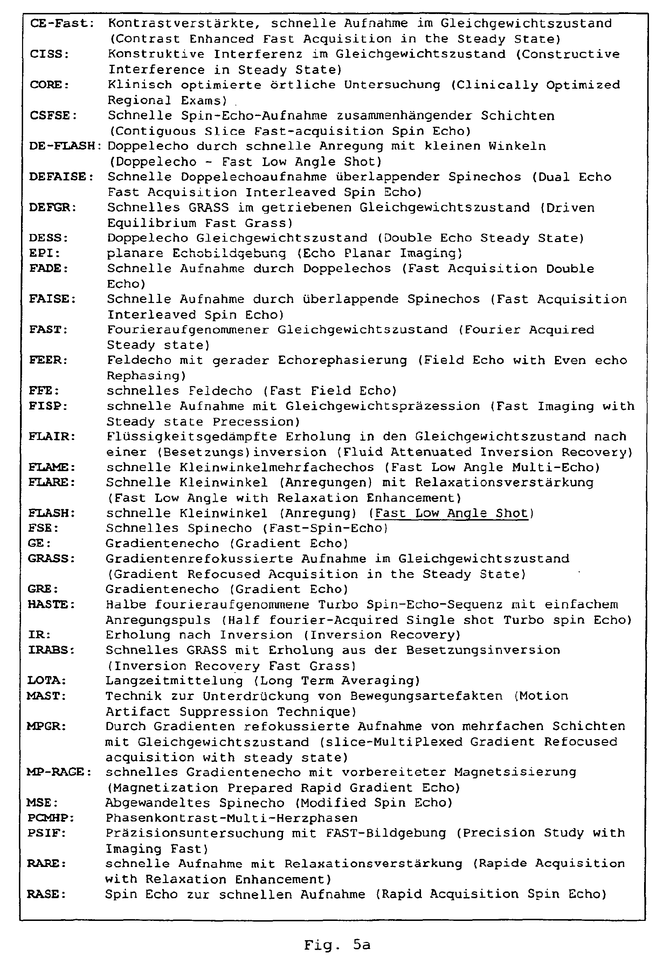

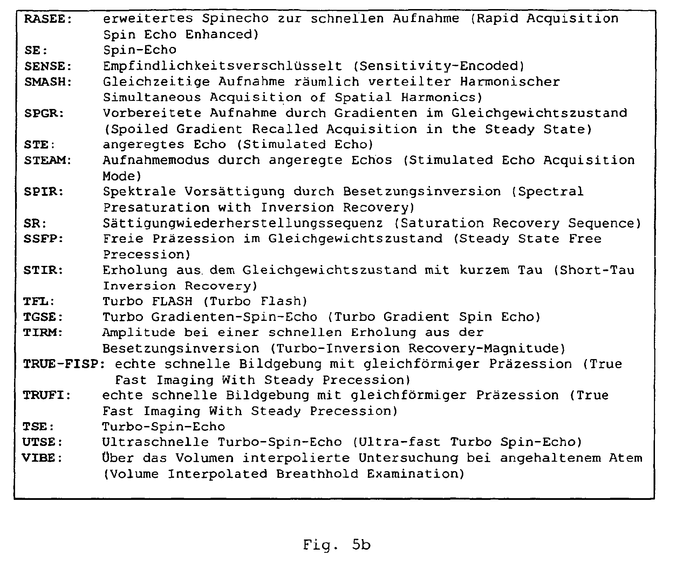

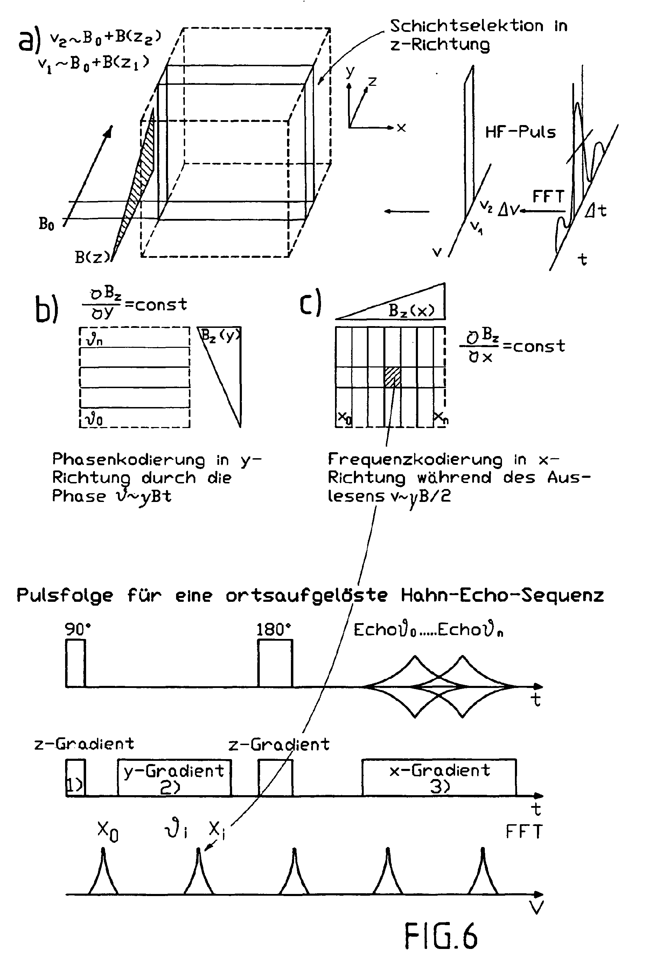

Bei dem erfindungsgemäßen Verfahren können alle Arten von MR - Sequenzen verwendet werden, wie sie zum Teil beispielsweise in den Figuren 5a, 5b aufgeführt sind, die eine Liste gebräuchlicher MR - Sequenzen enthält. Bei dem Ausführungsbeispiel verwendete Sequenzen zeichnen sich durch ihre unterschiedlichen Empfindlichkeiten aus und werden je nach zu untersuchendem Parameter benutzt. Das bei der Durchführung des erfindungsgemäßen Verfahrens immer zugrunde liegende Grundprinzip ist in Figur 6 schematisch dargestellt. Über einen Gradienten in z-Richtung bzw. in Richtung des Hauptmagnetfeldes B₀ findet eine Schichtselektion statt, was im Teilbild a) der Figur 6 dargestellt ist. Über die Gradienten in y - und x - Richtung findet über die Phase und die Frequenz eine Punktselektion in der ausgewählten Schicht statt, was in den Teilbildern b) und c) der Figur 6 (FFT = Fast Fourier Transformation) dargestellt ist. Bei den nachstehend wiedergegebenen Ausführungsbeispielen werden eine so genannte EPI-Sequenz (Echo-Planar-Imaging-Sequenz), eine T₂ - gewichtete Sequenz und eine T₁ - gewichtete Sequenz verwendet. Unter einer gewichteten Sequenz wird eine Pulsfolge verstanden, mit der der dynamische Bereich der jeweiligen Relaxationszeiten hervorgehoben wird, während der Rest unterdrückt wird. Es ist aber möglich, mit allen geläufigen oder gebräuchlichen MR - Sequenzen zu arbeiten.

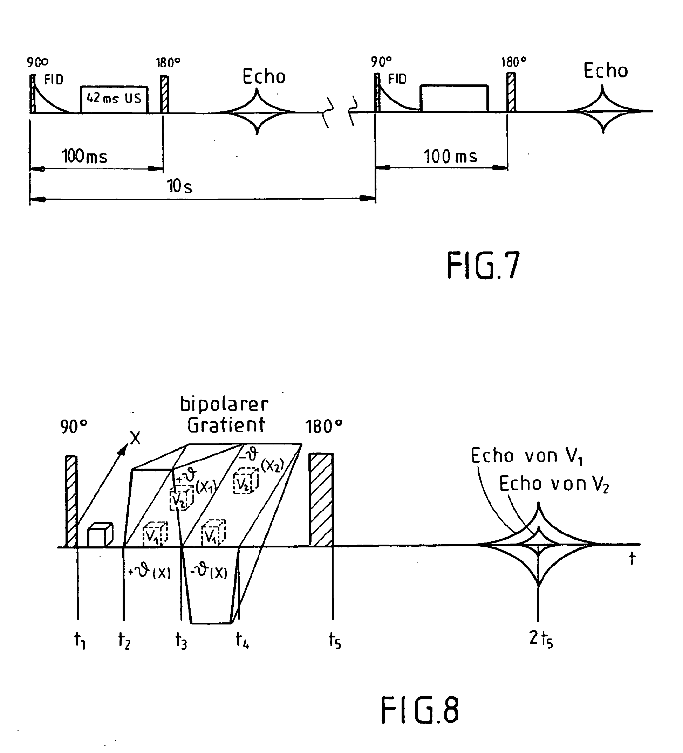

Der Zeitpunkt für den Ultraschallpuls wird bei den einzelnen Verfahrensdurchführungsbeispielen so gewählt, dass jedes Mal nach der jeweiligen Schichtauswahl in z-Richtung (nach dem 90°-Puls) Ultraschall eingestrahlt wird, wie dies schematisch in Figur 7 dargestellt (FID = Free Induction Decay) ist. Die Figur 6 zeigt die ortskodierenden Schritte in einer Hahn-Echo-Sequenz, wobei a) die Kodierung in z-Richtung durch selektive Anregung mit einem sinc-modelierten HF-Puls und damit die Festlegung der Ebene zeigt, b) die Phasenkodierung der γ-Richtung über den Feldgradient in dieser Richtung und damit die Festlegung der Zeile in der Ebene zeigt sowie c) die Frequenzkodierung in x-Richtung und damit die Festlegung eines Feldes in der Ebene bzw. dem Volumenelement oder Voxel zeigt, die durch einen x-Gradient bei der Auslese in dieser Richtung stattfindet.

Um nicht nur eine ortsaufgelöste Messung der Protonendichte mit den zugehörigen Relaxationsgrößen durchzuführen, sondern auch noch sensitiv auf Bewegung in der Probe zu sein, wird die benutzte Echosequenz um einen weiteren bipolaren Feldgradienten zwischen dem 90° und dem 180°-Puls erweitert, wie dies in Figur 8 dargestellt ist (Standardsequenz in der Tomographie). Diese zeigt den Einfluss der Bewegung auf das Echo unter Einfluss eines bipolaren Gradienten. Zwei Volumenelemente V₁ und V₂ sind mit ihrer Phase in Bezug zur Umgebung dargestellt. Das Volumenelement V₁ ist stationär und das Volumenelement V₂ bewegt sich in der x-Richtung. Rechts ist in der Figur 8 das zu erwartende Echosignal aus den beiden Volumenelementen (Voxeln) V₁ und V₂ qualitativ dargestellt.

Der weitere bipolare Feldgradient bewirkt, dass die Protonen in Abhängigkeit vom Gradienten und ihres Ortes einmal eine etwas höhere Frequenz und einmal eine etwas kleinere Frequenz aufweisen. Befinden sich die Protonen in Ruhe, bewirkt diese Schwankung in der Summe nichts. Bewegen sich die Protonen, erfahren sie eine nicht symmetrische Schwankung und erhalten auf diese Weise eine zusätzliche Phase, welche zu einer verminderten Signalamplitude im Echosignal führt, wie dies in Figur 8 für das bewegte bzw. sich bewegende Volumenelement V₂ im rechten Bildteil dargestellt ist.

Bei der Durchführung des erfindungsgemäßen Verfahrens am/im Kernspintomographen 1 bzw. in der MagnetresonanztomographieAnlage wurden drei Messreihen durchgeführt. Verwendung fanden eine diffusionssensitive Sequenz, mit der eine Kollektivbewegung dargestellt werden kann, sowie eine T₂- bzw. T₁- gewichtete Sequenz zur Darstellung des Einflusses auf die Relaxationszeiten. Vor jeder Messung wird zur Positionierung der Aufnahmeschichten eine sogenannte "Localizer-Sequenz" durchgeführt. Einen Ausschnitt aus einer mit einer Localizer-Sequenz erhaltenen Aufnahme zeigt die Figur 3 andeutungsweise in dem in dem Probenbehälter 11 vorhandenen Bildteil.

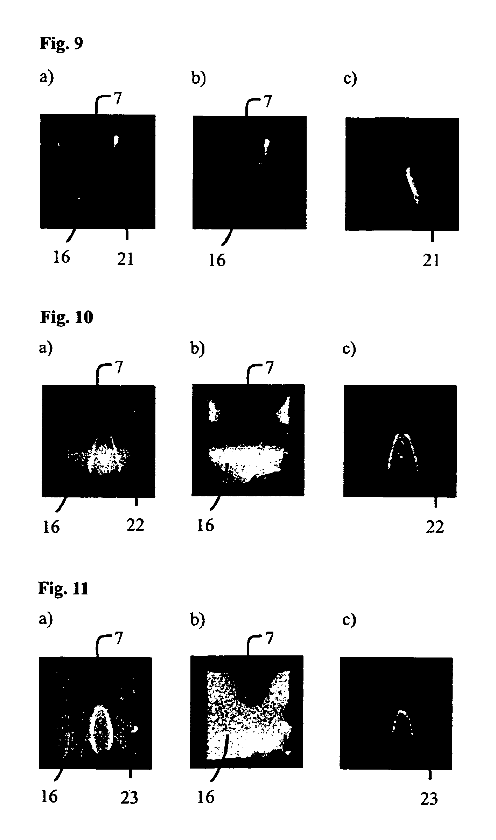

Die nachfolgenden Abbildungen 9 bis 12 zeigen jeweils Aufnahmen mit und ohne Ultraschalleinstrahlung in die bildgebende Sequenz oder Pulsfolge des Kernspintomographen 1. Die den jeweiligen Teilbildern a), b) und c) jeweils zu Grunde liegenden Messungen wurden unmittelbar hintereinander durchgeführt, ohne die Probe zu verändern. Der einzige Unterschied zwischen den den jeweiligen Teilbilden a) und b) jeweils zu Grunde liegenden Messungen ist lediglich der, dass einmal erfindungsgemäß mit einer an den Ultraschalltransmitter angelegten Spannung und einmal ohne Ultraschalleinwirkung gearbeitet wurde. In den Grauwertbildern sind in den jeweiligen Teilbildern a) und b) sich in Ruhe befindende Bereiche hell und bewegte Bereiche dunkel dargestellt. Es sind in den jeweiligen Teilbildern a) und b) jeweils die in der Figur 3 innerhalb des Probenbehälters 11 angeordneten Bereiche der untersuchten Flüssigkeit (Wasser) dargestellt.

Die Ergebnisse, die bei einer ersten, mit einer Gradienten-Echo-EPI-Sequenz in dem Kernspin- oder Magnetresonanztomographen 1 durchgeführt wurden, zeigt die Figur 9. Die linke Abbildung a) zeigt eine bei Durchführung des erfindungsgemäßen Verfahrens, also unter Anwendung von synchron in die Bildsequenz des Kernspintomographen eingestrahltem Ultraschall, erhaltene Darstellung. In diesem Fall wurde der bipolare Feldgradient in Richtung der Längsachse des Wellenleiters 7 gelegt, also in Schallausbreitungsrichtung. Die angelegte Spannung beträgt 50 Vₛₛ, was in Wasser (H₂O) einem Schalldruck von kleiner 1 bar entspricht (gemessen über die Beugung von sichtbarem Licht an der Ultraschallwelle in Wasser). Die verwendete Frequenz beträgt 9,45 MHz. Der Kernspintomograph 1 misst bei einem konstanten Magnetfeld von 1,5 T, was einer Lamorfrequenz von etwa 63 MHz entspricht. Die Gradienten betragen zwischen 5 und 20 mT pro Meter. Die Bildauflösung, die mit einem Gradienten in z-Richtung der Stärke 10 - 20 mT und einer HF-Pulslänge von 1-2 ms zu erreichen ist, liegt im submillimeter Bereich, die Bewegungsauflösung im Bereich von µm. Die Aufnahmen der Figuren 9 a), 9 b) und 9 c) wurden mit einer bewegungsempfindlichen NMR-Sequenz erhalten. Der in Figur 9 a) gezeigten Bilddarstellung liegt eine erfindungsgemäße Ultraschalleinstrahlung für 30 ms mit einer Anregungsspannung von 40 Vₛₛ in die Probe zugrunde. Die Figur 9 a) zeigt unterhalb des unteren Endes des dunkel dargestellten Wellenleiters 7 in der hell dargestellten Flüssigkeit 16 einen dunklen Streifen oder Kanal 21. Dieser Kanal 21 gibt in Form eines Kontrastunterschieds in der Darstellung des Bildes 9 a) den Bereich bewegter weicher Materie, in diesem Fall des bewegten Wassers, wieder. Die durch den eingestrahlten und eingekoppelten Ultraschall bewirkte induzierte Bewegung führt zu der dunkleren Bilddarstellung dieses Bereiches. Es wird damit der Schallweg oder der Ultraschallstrahlungsdruck oder der ultraschallinduzierte Impulsübertrage als Kontrastunterschied in der Abbildung 9 a) sichtbar und erkennbar gemacht. Unter dem Schallemitter 2 bzw. dem Wellenleiter 7 bildet sich der Kanal 21 aus, der auf eine Bewegung entlang des angelegten bipolaren Gradienten zurückzuführen ist. Ein Gradient in der zu dieser Ausbreitungsrichtung senkrechten Achse zeigt keinen messbaren und/oder bildlich darstellbaren und erkennbaren Unterschied, da in dieser Richtung keine Bewegung durch den in das Hochfrequenzpulssignalfeld des Kernspintomographen 1 eingestrahlten Ultraschall erzeugt wird.

Die Abbildung 9 b) wurde ohne Ultraschalleinstrahlung, aber unter ansonsten gleichen Bedingungen aufgenommen. Es zeigt sich keine Bewegung in der hell dargestellten Flüssigkeit 16 und folglich auch kein Kanal 21. Die rechte Abbildung 9 c) zeigt die die Differenz der Grauwertbilder 9 a) und 9 b) und folglich ausschließlich den Kanal 21.

Darüber hinaus wurden ferner auch noch nicht dargestellte Messungen an Flüssigkeiten mit unterschiedlicher Viskosität durchgeführt, woraus sich ergibt, dass die Reichweite des Ultraschalleinflusses mit zunehmender Viskosität sinkt, während die Intensität mit steigender Viskosität aufgrund der in der Regel mit der Viskosität ansteigenden Reibung zunimmt.

Die Figur 10 zeigt Darstellungen, die mit einer auf die T₂ - Zeit sensitiven Sequenz erhalten werden. Auch in diesem Fall ist in der Abbildung 10 a) bei der Anwendung von Ultraschall eine Struktur 22 unter dem Schallkopf 13 bzw. unterhalb des Wellenleiters 7 zu erkennen. Die Figur 10 zeigt Messergebnisse, welche mit einer T₂ - gewichteten Sequenz in Wasser 16 erhalten werden. Während der der Abbildung 10 a) zugrunde liegenden Sequenz wird Ultraschall synchronisiert eingestrahlt. Die Abbildung 10 b) ist wiederum ohne Ultraschalleinwirkung aufgenommen worden und die rechte Abbildung 10 c) zeigt wiederum die Differenz der Grauwertbilder 10 a) und 10 b).

Die Figur 11 zeigt Abbildungen, die mit einer auf die T₁ - Zeit sensitiven Sequenz erhalten werden. Auch hier bildet sich ähnlich zu den T₂ - Messungen eine Struktur 23 aus, wie aus der Abbildung 11 a) ersichtlich ist, die das Ergebnis einer T₁ - gewichtete Messung an Wasser (H₂0) unter Ultraschalleinfluss und die Abbildung 11 c) die Differenz der Grauwertbilder 11 a) und 11 b).

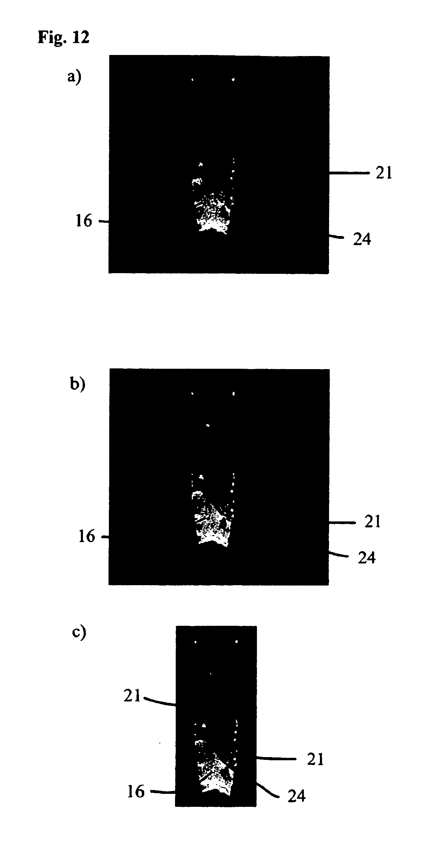

Schließlich zeigen die weiteren Figuren 12 a) bis 12 c) nochmals den Einfluss des Ultraschalls und die Möglichkeit, den dadurch in einer weichen Materie, bzw. im vorliegenden Fall Wasser, erzeugten viskosen Impulsübertrag in der Bilddarstellung eines Kernspintomographen 1 in Form einer Kontrastausbildung als Streifen 21 oder Kanal 21 sichtbar zu machen. Hierbei ist in der Flüssigkeit 16 eine Glasscheibe 24 angeordnet, an welcher die Flüssigkeitsbewegung oder der sichtbar gemachte Schallstrahlungsdruck nach den Gesetzen der Optik (Einfallwinkel gleich Ausfallwinkel) abgelenkt wird. Je nach Richtung des oder der (Feld)Gradienten ist entweder der auf die Glasscheibe 24 einfallende (Figur 12 a) oder der ausfallende (Figur 12 b) Schallweg als dunkler Streifen 21 oder sind beide Schallwege (Figur 12 c) als abgewinkelt verlaufender dunkler Streifen 21 in dem hellen Probenmaterial 16 kontrastreich und sichtbar in der Bilddarstellung des Kernspintomographen 1 erkennbar.

Durch die Verwendung des Kernspintomographen 1 kann somit in der Bilddarstellung von Kernspin- oder Magnetresonanztomographen kontrastreich dargestellt werden, dass durch den Ultraschall eine Kollektivbewegung in die Probe aus weicher Materie 16 eingebracht wird und ein schallinduzierter viskoser Impulsübertrag zwischen den Materieteilchen ausgelöst wird. Es wird der Schallweg in der weichen Materie oder Flüssigkeit bzw. der Ultraschallstrahlungsdruck in der dem Signalfeld der Magnetresonanztomographie- bzw. Kernspintomographie - Anlage einerseits und dem Ultraschallsignalfeld andererseits ausgesetzten weichen Materie 16 kontrastreich dargestellt. Ortsaufgelöste Messungen an verschieden viskosen Flüssigkeiten bestätigen weiterhin, dass die Viskosität die Reichweite und Art des Ultraschalleinflusses stark ändert. Darüber hinaus zeigen Messungen mit in der Medizin angewendeten Messsequenzen (T₁-, T₂-gewichtet) eine durch den Ultraschall hervorgerufene Struktur 22, 23. Damit kann durch gezieltes Einstrahlen von Ultraschall während einer NMR-Messung das Spektrum der Kontrastmöglichkeiten zum Beispiel um den Parameter Viskosität erweitert werden.

Die gefundene Methode kann zur Diagnostik bei medizinischen Untersuchungen am Menschen genutzt werden, da die bei dem erfindungsgemäßen Verfahren in ein Medium (weiche Materie) eingebrachte Energie weit unter den erlaubten Grenzwerten liegt. Die Druckamplitude P liegt bei ungefähr 1 bar, was nach heutigem Stand unbedenklich ist.

Mit der Erfindung kann das Prinzip der Kombination von Ultraschall und NMR (Nuclear magnetic resonance) umgesetzt werden. Durch eine genaue Positionierung des Ultraschalls, können Areale weicher Materie selektiv dem Ultraschall ausgesetzt werden und so Anomalitäten genauer charakterisiert und spezifiziert werden. Da der Schall auf Dichteschwankungen sehr empfindlich reagiert (Dämpfung, Streuung), ist es möglich mit dem erfindungsgemäßen Verfahren Substrukturen, welche von der bisherigen Kernspintomographie und Ultraschalldiagnostik nicht aufgelöst werden können, sichtbar zu machen.

Auch ist es möglich, die in eine Flüssigkeit eingebrachte Dynamik zu nutzen und/oder eine periodische Bewegung in der Flüssigkeit zu nutzen, um damit gezielt eingebrachte Partikel (z.B. Kontrastmittel) zu bewegen. Damit besteht eine weitere Kopplungsmöglichkeit mit Einflussnahme auf das Kernspinsystem während einer NMR(Nuclear magnetic resonance)- Messung.

Aufgrund der Tatsache, dass in allen organischen Stoffen viele Protonen vorhanden sind, besteht mit dem erfindungsgemäßen Verfahren die Möglichkeit, die Protonen in definierter Weise zu manipulieren und damit die Empfindlichkeit der bekannten Parameter zu steigern und auch neue, wie die Viskosität, hinzu zu gewinnen.

Ultraschall (Frequenz zwischen 2 x 10⁴ bis 1 x 10⁹ Hz) ist das hierfür geeignete Werkzeug. Bei Frequenzen im Megaherzbereich liegt die Wellenlänge für Schall in Flüssigkeiten im Bereich von einigen zehntel Millimetern und kann somit sehr gezielt zur lokalen Manipulation eingesetzt werden. Eine weitere positive Eigenschaft ist die Verträglichkeit von Ultraschall für menschliche und tierische Zellen, solange die einwirkende Leistung gering gehalten wird.

Die Differenz der Magnetisierungen bzw. der Signalamplituden zwischen Messungen mit und ohne Schall enthalten ein Maß für die Stärke des Ultraschalleinflusses. Hierbei lässt sich für die Fälle des Gleichgewichtes und des Ungleichgewichtes ein Einfluss des Ultraschalls in der Gesamtkernmagnetisierung eines Probenkörpers wieder finden.

Longitudinaler Schall ist eine Druckschwankung, die sich in einem Medium räumlich ausbreitet. Der zeitliche Verlauf an einem Raumpunkt liefert die für den Schall bzw. eine Schallwelle charakteristischen Größen. Dies sind die Bewegungsamplitude, die Geschwindigkeitsamplitude, die Druckamplitude, die Schallgeschwindigkeit, die Schallkennimpedanz, die Schalldämpfung, die angibt, nach welcher Strecke die Druckamplitude durch Energieabgabe auf ein e-tel abgefallen ist, und der Schallstrahlungsdruck, wobei es sich um den Überdruck handelt, der im Mittel in einem von einer Schallwelle durchstrahlten Medium herrscht. Mit Hilfe der Fouriertransformation kann jeder Druckschwankung über ihren zeitlichen Verlauf ein Frequenzspektrum zugeordnet werden. Jede Druckschwankung kann durch eine Superposition aus amplitudenmodulierten periodischen Schwingungen dargestellt werden. Damit können die Gesetzte der Wellenmechanik auf die Ausbreitung von Schall angewendet werden, weshalb von Schallwellen gesprochen wird. Zur Erzeugung von Schallwellen eignen sich insbesondere Piezokeramiken.

In einer weichen Materie, worunter biologisches Gewebe, insbesondere aber auch wasserhaltige Flüssigkeiten verstanden werden, befinden sich die Nukleonen nicht mehr an einem festen Ort. Die Position eines Nukleons kann nur noch statistisch beschrieben werden, so dass in derartigen Medien oder Materien eine Überlagerung der individuellen Nukleonenbewegung und der sich durch das jeweilige Medium bewegenden Dichteschwankung betrachtet werden. Im Wesentlichen besteht der Einfluss des Ultraschalls auf das Kernspinensemble zum einen in der lokalen Druckänderung und zum anderen in einer in das Medium/die weiche Materie eingebrachten Kollektivbewegung.

Der Druckunterschied nimmt Einfluss auf die mittlere Stoßfrequenz und die Abstände der Kerne untereinander. Auf diese Art werden die Relaxationszeiten T₁ und T₂ beeinflusst. Die Kollektivbewegung führt zu einer Positionsänderung in der Probe bzw. im Magnetfeld, wodurch Einfluss auf die T*₂-Zeit analog zur Diffusion genommen wird, wenn Magnetfeldinhomogenitäten vorhanden sind. Die Bewegung im durchstrahlten Medium entsteht durch die Dämpfung, bei der es zu einem insbesondere viskosen Impulsübertrag kommt. Dieser führt zu einer Bewegung längs der Ausbreitungsrichtung der Schallwelle. Die Reichweite der kollektiven Bewegung hängt von der Viskosität der Flüssigkeit ab. Bei sehr hoher Viskosität wird durch die hohe Anzahl an Stößen der übertragene Impuls schneller diffus als bei niedriger Viskosität, bei der der Impuls länger seine Richtung beibehält.

Durch die Beaufschlagung mit Ultraschall werden das Kernspinensemble und die Magnetisierung der jeweiligen weichen Materie beeinflusst. Der Ultraschalleinfluss dürfte maximal sein, wenn die Frequenz des eingestrahlten Ultraschalls genau mit der Lamorfrequenz übereinstimmt.

Eine Messsequenz mit der der gesamte Einfluss des Ultraschalls auf die Magnetisierung festgestellt und bestimmt werden kann, ist die Hahn-Echo-Sequenz. Der Einfluss auf die Dephasierungsgeschwindigkeit spiegelt sich in der Linienform des Frequenzspektrums wider, während der Einfluss auf Relaxationszeiten T₂ und T₁ sich kombiniert in der Signalamplitude widerspiegelt. Der Zeitpunkt für das Einstrahlen des Ultraschalls liegt zwischen dem 90°- und 180°-Puls. Ob der Ultraschall vor oder nach dem 180°-Puls eingestrahlt wird, macht keinen Unterschied. Ungünstig wäre eine Einstrahlung symmetrisch um den 180°-Puls, da sich so möglicherweise durch den Inversionspuls Effekte aufheben könnten.

Bei den beschriebenen Verfahrensdurchführungen wird über die Hahn-Echo-Sequenz das Echo aufgezeichnet. Der Ultraschall wird zwischen dem 90° und dem 180°Puls eingestrahlt. Der Ultraschall wird beispielsweise 5 ms nach dem 90°-Puls eingestrahlt und der Probenkörper bzw. Probenbehälter 11 wird den mechanischen Schwingungen ausgesetzt. Die Anregungsspannung an der Piezokermik 14 beträgt beispielsweise 40 Vₛₛ und die Betriebszeit des Transmitters beträgt beispielsweise 50 ms.

## Patentansprüche

1. Verfahren zur Erzeugung eines Kontrast gebenden Effektes in der Bilddarstellung eines Magnetresonanztomographie-Verfahrens und/oder -systems, bei welchem weiche Materie (16) dem Einfluss eines Hochfrequenzpulsfeldes einer Magnetresonanztomographieanlage ausgesetzt und in das Hochfrequenzpulssignalfeld Ultraschallsignale gepulst eingekoppelt und eingestrahlt werden,
**dadurch gekennzeichnet,**
**dass** eine Ultraschallsignalpulssequenz derart in eine von der Magnetresonanztomographieanlage erzeugte Bildpulsfolge oder -sequenz eingekoppelt und synchronisiert eingestrahlt wird, dass in der Bilddarstellung der Magnetresonanztomographieanlage die durch Ultraschallstrahlungsdruck induzierte Bewegung der weichen Materie (16) als Bildkontrast (21) darstellbar und/oder dargestellt wird.

2. Verfahren nach Anspruch 1, **dadurch gekennzeichnet, dass** die Sequenzen der Hochfrequenzpulse diffusionssensitive Sequenzen sind.

3. Verfahren nach einem der vorhergehenden Ansprüche, **dadurch gekennzeichnet, dass** die Bildpulsfolge oder - sequenz eine Hahn-Echo-Sequenz ist, die um einen bipolaren Feldgradienten, insbesondere zwischen dem 90°- und dem 180°-Puls erweitert wird.

4. Verfahren nach einem der vorhergehenden Ansprüche, **dadurch gekennzeichnet, dass** die Ausbreitungsrichtung des Ultraschalls parallel zum diffusionssensitiven Gradienten gewählt wird.

5. Verfahren nach einem der vorhergehenden Ansprüche, **dadurch gekennzeichnet, dass** längs eines Gradienten, vorzugsweise eines Gradienten in Richtung des Hauptmagnetfeldes B₀, eine Schicht selektiert und in der selektierten Schicht in den verbleibenden Richtungen, vorzugsweise in y- und x-Richtung, über die Phase und die Frequenz eine Punktselektion durchgeführt wird, wobei der Ultraschallpuls jeweils nach der Schichtselektion eingestrahlt wird.

6. Verfahren nach einem der vorhergehenden Ansprüche, **dadurch gekennzeichnet, dass** Ultraschall mit einer Frequenz zwischen 2 und 30 MHz und einer Pulslänge zwischen 1 und 50 ms und einem Schalldruck von ≤ 1 bar eingekoppelt und eingestrahlt wird.

7. Verfahren nach einem der vorhergehenden Ansprüche, **dadurch gekennzeichnet, dass** ein abgeschirmter Ultraschallemitter (2) und/oder Ultraschalltransmitter innerhalb der zentralen Öffnung eines Magnetresonanztomographen (1) angeordnet und in dessen Hochfrequenzpulsfeld eingekoppelt wird.

8. Verfahren nach einem der vorhergehenden Ansprüche, **dadurch gekennzeichnet, dass** Ultraschall über einen Wellenleiter (7) in das Hochfrequenzpulsfeld der Magnetresonanztomographieanlage eingekoppelt wird.

9. Verfahren nach einem der vorhergehenden Ansprüche, **dadurch gekennzeichnet, dass** der Ultraschallemitter (2) und/oder Ultraschalltransmitter mittels einer HF-Antenne (4) mit dem Hochfrequenzpulsfeld synchronisiert gekoppelt wird.

10. Verfahren nach Anspruch 9, **dadurch gekennzeichnet, dass** mit der HF-Antenne (4) das HF-Pulssignal des Magnetresonanztomographen (1) detektiert und für die gezielte Einstrahlung des Ultraschallpulses genutzt wird.

11. Verfahren nach einem der vorhergehenden Ansprüche, **dadurch gekennzeichnet, dass** zur zeitlichen Positionierung des Ultraschalls in der bildgebenden Sequenz ein Steuersignal der Magnetresonanztomographieanlage genutzt wird.

12. Verfahren nach einem der vorhergehenden Ansprüche, **dadurch gekennzeichnet, dass** der Ultraschallemitter (2) und/oder Ultraschalltransmitter im Bereich einer Hochfrequenzspule (8a) innerhalb des Magnetresonanztomographen (1) angeordnet wird oder der Ultraschall mittels eines Wellenleiters in diesen Bereicht transportiert wird.

13. Verwendung einer Magnetresonanztomographieanlage durch Einbringung eines abgeschirmten Ultraschallemitters (2) in die zentrale Öffnung eines Magnetresonanztomographen (1) und Einkopplung des Ultraschallemitters (2) in das Hochfrequenzpulsfeld des Magnetresonanztomographen (1) sowie zeitlich abgestimmtes Einstrahlen von Ultraschallpulsen in das von der Magnetresonanztomographieanlage erzeugte Hochfrequenzpulsfeld zur Erzeugung einer kontrastreichen Darstellung der in einer in das Untersuchungsfeld des Magnetresonanztomographen (1) eingebrachten weichen Materie (16) erzeugten, durch Ultraschallstrahlungsdruck induzierten Bewegung der Materie (16) in der Bilddarstellung der Magnetresonanztomographieanlage.

14. Magnetresonanztomographieanlage, umfassend einen abgeschirmten Ultraschallemitter (2) mit Mitteln zur Detektion eines bildgebend genutzten Hochfrequenzpulsfeldes eines mit dem Ultraschallemitter (2) in Wirkverbindung stehenden Magnetresonanztomographen (1) und mit Mitteln zur synchronisierten Einstrahlung von Ultraschallpulssequenzen in dessen Bildpulsfolge oder - sequenz sowie Mitteln zur Darstellung einer gerichteten, durch Ultraschallstrahlungsdruck induzierten Bewegung in einem in dem Magnetresonanztomographen (1) der Magnetresonanztomographieanlage dem Hochfrequenzpulsfeld ausgesetzten Volumenelement aus weicher Materie (16).

## Claims

1. Method for generating a contrast-producing effect in the image of a magnetic resonance tomography method and/or system, in which soft matter (16) is exposed to the influence of a high-frequency pulse field of a magnetic resonance tomography unit and ultrasound signals are coupled into and irradiate the high-frequency pulse-signal field in a pulsed manner, **characterised in that** an ultrasound signal pulse sequence is coupled into and synchronously irradiates an image pulse train or an image pulse sequence, generated by the magnetic resonance tomography unit, in such a way that the movement induced in the soft matter (16) by the ultrasound radiation pressure can be depicted, or is depicted, as an image contrast (21) in the image of the magnetic resonance tomography unit.

2. Method according to claim 1, **characterised in that** the sequences of the high-frequency pulses are diffusion-sensitive sequences.

3. Method according to any of the preceding claims, **characterised in that** the image pulse train or image pulse sequence is a Hahn echo sequence which extends about a bipolar field gradient, in particular between the 90° and the 180° pulse.

4. Method according to any of the preceding claims, **characterised in that** the direction of propagation of the ultrasound is selected so as to be parallel to the diffusion-sensitive gradient.

5. Method according to any of the preceding claims, **characterised in that** a layer is selected along a gradient, preferably along a gradient in the direction of the main magnetic field B₀, and point selection is carried out in the remaining directions, preferably in the y and x direction, in the selected layer via the phase and the frequency, the ultrasound pulse being irradiated after layer selection in each case.

6. Method according to any of the preceding claims, **characterised in that** ultrasound is coupled in and irradiated at a frequency of between 2 and 30 MHz, at a pulse duration of between 1 and 50 ms and at a sound pressure of ≤ 1 bar.

7. Method according to any of the preceding claims, **characterised in that** a shielded ultrasound emitter (2) and/or ultrasound transmitter is arranged within the central opening of a magnetic resonance tomograph (1) and coupled into the high-frequency pulse field thereof.

8. Method according to any of the preceding claims, **characterised in that** ultrasound is coupled via a waveguide (7) into the high-frequency pulse field of the magnetic resonance tomography unit.

9. Method according to any of the preceding claims, **characterised in that** the ultrasound emitter (2) and/or ultrasound transmitter is coupled synchronously to the high-frequency pulse field by means of a HF-antenna (4).

10. Method according to claim 9, **characterised in that** the HF-pulse signal of the magnetic resonance tomograph (1) is detected with the HF-antenna (4) and used for the targeted irradiation of the ultrasound pulse.

11. Method according to any of the preceding claims, **characterised in that** a control signal of the magnetic resonance tomography unit is used for temporally positioning the ultrasound in the image-producing sequence.

12. Method according to any of the preceding claims, **characterised in that** the ultrasound emitter (2) and/or ultrasound transmitter is arranged in the region of a high-frequency coil (8a) within the magnetic resonance tomograph (1), or the ultrasound is transported to this region by means of a waveguide.

13. Use of a magnetic resonance tomography unit by placing a shielded ultrasound emitter (2) in the central opening of magnetic resonance tomograph (1), by coupling the ultrasound emitter (2) into the high-frequency pulse field of the magnetic resonance tomograph (1), and by means of timed irradiation of ultrasound pulses into the high-frequency pulse field, generated by the magnetic resonance tomography unit, for generating a high-contrast depiction, in the image of the magnetic resonance tomography unit, of the movement of the matter (16) which is generated in soft matter (16) placed in the examination field of the magnetic resonance tomograph (1) and which is induced by ultrasound radiation pressure.

14. Magnetic resonance tomography unit comprising a shielded ultrasound emitter (2) having means for detecting a high-frequency pulse field, used to produce an image, of a magnetic resonance tomograph (1) which is operatively connected to the ultrasound emitter (2), and having means for the synchronised irradiation of ultrasound pulse sequences in the image pulse train or image pulse sequence thereof, and means for depicting a directed movement, induced by ultrasound radiation pressure, in a volume element which is made of soft matter (16) and exposed to the high-frequency pulse field in the magnetic resonance tomograph (1) of the magnetic resonance tomography unit.

## Revendications

1. Procédé de production d'un effet formant contraste dans la représentation de l'image d'un procédé et/ou d'un système de tomographie par résonance magnétique, dans lequel une matière douce (16) est exposée à l'influence d'un champ d'impulsions haute fréquence d'une installation de tomographie par résonance magnétique, et des signaux ultrasons sont injectés et envoyés de manière pulsée dans le champ de signaux d'impulsions haute fréquence,
**caractérisé en ce que**
une séquence d'impulsions de signaux ultrasons est injectée, et envoyée de manière synchronisée, dans une suite ou une séquence d'impulsions d'images produite par l'installation de tomographie par résonance magnétique, de telle sorte que, dans la représentation de l'image de l'installation de tomographie par résonance magnétique, le mouvement de la matière douce (16), induit par la pression du rayonnement ultrason, soit représentable et/ou représenté en tant que contraste d'image (21).

2. Procédé selon la revendication 1, **caractérisé en ce que** les séquences des impulsions haute fréquence sont des séquences sensibles à la diffusion.

3. Procédé selon l'une des revendications précédentes, **caractérisé en ce que** la suite ou la séquence d'impulsions d'images est une séquence d'échos de Hahn, qui est élargie d'un gradient de champ bipolaire, en particulier d'entre l'impulsion 90° et l'impulsion 180°.

4. Procédé selon l'une des revendications précédentes, **caractérisé en ce que** la direction de propagation des ultrasons est choisie parallèle au gradient sensible à la diffusion.

5. Procédé selon l'une des revendications précédentes, **caractérisé en ce que**, le long d'un gradient, de préférence d'un gradient dans la direction du champ magnétique principal B₀, une couche est sélectionnée, et une sélection de points est mise en oeuvre dans la couche sélectionnée, dans les directions restantes, de préférence dans les directions y et x, via la phase et la fréquence, dans lequel l'impulsion ultrason est respectivement envoyée après la sélection de la couche.

6. Procédé selon l'une des revendications précédentes, **caractérisé en ce que** sont injectés et envoyés des ultrasons avec une fréquence comprise entre 2 et 30 MHz et avec une longueur d'impulsion comprise entre 1 et 50 ms et avec une pression acoustique ≤ 1 bar.

7. Procédé selon l'une des revendications précédentes, **caractérisé en ce qu'**un émetteur d'ultrasons blindé (2) et/ou un transmetteur d'ultrasons est agencé à l'intérieur de l'ouverture centrale d'un tomographe par résonance magnétique (1), et est injecté dans le champ d'impulsions haute fréquence de ce dernier.

8. Procédé selon l'une des revendications précédentes, **caractérisé en ce que** les ultrasons sont injectés, via un guide d'ondes (7), dans le champ d'impulsions haute fréquence de l'installation de tomographie par résonance magnétique.

9. Procédé selon l'une des revendications précédentes, **caractérisé en ce que** l'émetteur d'ultrasons (2) et/ou le transmetteur d'ultrasons sont couplés avec le champ d'impulsions haute fréquence, de manière synchronisée et via une antenne HF (4).

10. Procédé selon la revendication 9, **caractérisé en ce que**, à l'aide de l'antenne HF (4), le signal d'impulsions HF du tomographe par résonance magnétique (1) est détecté et utilisé pour l'envoi ciblé de l'impulsion ultrason.

11. Procédé selon l'une des revendications précédentes, **caractérisé en ce que**, pour le positionnement temporel des ultrasons dans la séquence de représentation de l'image, on utilise un signal de commande de l'installation de tomographie par résonance magnétique.

12. Procédé selon l'une des revendications précédentes, **caractérisé en ce que** l'émetteur d'ultrasons (2) et/ou le transmetteur d'ultrasons est agencé dans la zone d'une bobine haute fréquence (8a) à l'intérieur du tomographe par résonance magnétique (1), ou les ultrasons sont transportés dans cette zone au moyen d'un guide d'ondes.

13. Utilisation d'une installation de tomographie par résonance magnétique, par introduction d'un émetteur d'ultrasons blindé (2) dans l'ouverture centrale d'un tomographe par résonance magnétique (1) et injection de l'émetteur d'ultrasons (2) dans le champ d'impulsions haute fréquence du tomographe par résonance magnétique (1), ainsi que par envoi, de manière définie dans le temps, d'impulsions ultrasons dans le champ d'impulsions haute fréquence produit par l'installation de tomographie par résonance magnétique, pour produire une représentation à fort contraste du mouvement de la matière (16) dans la représentation de l'image de l'installation de tomographie par résonance magnétique, lequel mouvement est produit dans une matière douce (16) introduit dans le champ d'examen du tomographe par résonance magnétique (1) et induit par la pression du rayonnement ultrason.

14. Installation de tomographie par résonance magnétique comprenant un émetteur ultrason blindé (2), avec des moyens pour la détection d'un champ, utilisé pour former une image, d'impulsions haute fréquence d'un tomographe par résonance magnétique (1) situé en liaison fonctionnelle avec l'émetteur d'ultrasons (2), et avec des moyens pour un envoi synchronisé de séquences d'impulsions ultrasons dans sa suite ou sa séquence d'impulsions d'images, ainsi que des moyens pour représenter un mouvement orienté, induit par la pression de rayonnement ultrason, dans un élément de volume exposé, dans le tomographe par résonance magnétique (1) de l'installation de tomographie par résonance magnétique, au champ d'impulsions haute fréquence, cet élément étant constitué d'une matière douce (16).
